# EUROPEAN PATENT APPLICATION

(11) **EP 1 393 776 A1**
(43) Date of publication of application: **03.03.2004**
(21) Application number: 02078358.5
(22) Date of filing: 14.08.2002
(51) Int. Cl.: A61P 35/00, A61P 35/02, A61K 39/395, C07K 14/47

(54) **Use of genes identified to be involved in tumor development for the development of anti-cancer drugs and diagnosis of cancer**

(71) Applicant: Erasmus University Medical Center Rotterdam, 3015 GD Rotterdam (NL)
(72) Inventor: Touw, Ivo Paul, 3053 LE Rotterdam (NL); Delwel, Hendrik Rudolf, 3273 XD Westmaas (NL)

(57) **Abstract**

The invention relates to the use of inhibitors of the expressed proteins of the murine genes and/or their human homologues listed in Table 1 for the preparation of a therapeutical composition for the treatment of cancer, in particular for the treatment of solid tumors of lung, colon, breast, prostate, ovarian, pancreas and leukemia and the use of the genes listed in Table 1 for the diagnosis of cancer. The invention also relates to the therapeutical compositions comprising the inhibitors and to methods for development of the inhibitor compounds.

## Description

The present invention relates to the use of the murine genes identified by retroviral insertional tagging as well as their human homologues for the identification and development of anti-cancer drugs, like small molecule inhibitors, antibodies, antisense molecules, RNA interference (RNAi) molecules and gene therapies against these genes and/or their expression products, and especially anti-cancer drugs effective against solid tumors of e.g. lung, colon, breast, prostate, ovarian, pancreas and leukemia. The invention further relates to the use of said genesfor the diagnosis of cancer, the use of antibodies or fragments derived therefrom for the diagnosis of cancer, pharmaceutical preparations comprising one or more of said inhibitors and methods for the treatment of cancer using said pharmaceutical preparations.

After a quarter century of rapid advances, cancer research has generated a rich and complex body of knowledge revealing cancer to be a disease involving dynamic changes in the genome. Cancer is thought to result from at least six essential alterations in cell physiology that collectively dictate malignant growth: self-sufficiency in growth signals, insensitivity to growth-inhibitory (anti-growth) signals, evasion of programmed cell death (apoptosis), limitless replicative potential, sustained angiogenesis, and tissue invasion and metastasis.

In general, these essential alterations are the result of mutations in genes involved in controlling these cellular processes. These mutations include deletions, point mutations, inversions and amplifications. The mutations result in either an aberrant level, timing, and/or location of expression of the encoded protein or a change in function of the encoded protein. These alterations can affect cell physiology either directly, or indirectly, for example via signalling cascades.

Identifying genes which promote the transition from a normal cell into a malignant cell provides a powerful tool for the development of novel therapies for the treatment of cancer.

One of the most common therapies for the treatment of cancer is chemotherapy. The patient is treated with one or more drugs which function as inhibitors of cellular growth and which are thus intrinsically toxic. Since cancer cells are among the fastest growing cells in the body, these cells are severely affected by the drugs used. However, also normal cells are affected resulting in, besides toxicity, very severe side-effects like loss of fertility.

Another commonly used therapy to treat cancer is radiation therapy. Radiotherapy uses high energy rays to damage cancer cells and this damage subsequently induces cell cycle arrest. Cell cycle arrest will ultimately result in programmed cell death (apoptosis). However, also normal cells are irradiated and damaged. In addition, it is difficult to completely obliterate, using this therapy, all tumor cells. Importantly, very small tumors and developing metastases cannot be treated using this therapy. Moreover, irradiation can cause mutations in the cells surrounding the tumor which increases the risk of developing new tumors. Combinations of both therapies are frequently used and a subsequent accumulation of side-effects is observed.

The major disadvantage of both therapies is that they do not discriminate between normal and tumor cells. Furthermore, tumor cells have the tendency to become resistant to these therapies, especially to chemotherapy.

Therapies directed at tumor specific targets would increase the efficiency of the therapy due to i) a decrease in the chance of developing drug resistance, ii) the drugs used in these tumor specific therapies are used at much lower concentrations and are thus less toxic and iii) because only tumor cells are affected, the observed side-effects are reduced.

The use of tumor specific therapies is limited by the number of targets known. Since tumors mostly arise from different changes in the genome, their genotypes are variable although they may be classified as the same disease type. This is one of the main reasons why not a single therapy exists that is effective in all patients with a certain type of cancer. Diagnosis of the affected genes in a certain tumor type allows for the design of therapies comprising the use of specific anti-cancer drugs directed against the proteins encoded by these genes.

Presently, only a limited number of genes involved in tumor development are known and there is a clear need for the identification of novel genes involved in tumor development to be used to design tumor specific therapies and to define the genotype of a certain tumor.

In the research that led to the present invention, a number of genes were identified by proviral tagging to be involved in tumor development. Proviral tagging is a method that uses a retrovirus to infect normal vertebrate cells. After infection, the virus integrates into the genome thereby disrupting the local organization of the genome. This integration is random and, depending on the integration site, affects the expression or function of nearby genes. If a gene involved in tumor development is affected, the cell has a selective advantage to develop into a tumor as compared to the cells in which no genes involved in tumor development are affected. As a result, all cells within the tumor originating from this single cell will carry the same proviral integration. Through analysis of the region nearby the retroviral integration site, the affected gene can be identified. Due to the size of the genome and the total number of integration sites investigated in the present invention, a gene that is affected in two or more independent tumors must provide a selective advantage and therefore contribute to tumor development. Such sites of integration are designated as common insertion sites (CIS).

The genes claimed in the present invention are all identified by common insertion sites and are so far not reported to be involved in tumor development. The novel cancer genes that were identified in this manner are the following: *Adam11, AI462175, Cd24a, Edg3, Itgp, Kcnj16, Kcnk5, Kcnn4, Ltb, Ly108, Ly6i,* mouse homologue of *EMILIN, Mrc*1, *Ninj2, Nphs1, Sema4b, Tm9sf2,* and *Tnfrsf17*, encoding cell surface proteins; *Apobec2, Btd*, *Cds2, Clpx, Ddx19, Ddx21, Dnmt2, Dqx1, Hdac7a, Lce-pending, Mgat1,* mouse homologue of *CILP,* mouse homologue of *NOH61, Nudel-pending, Pah, Pdi1*, *Ppia, Prps1*, *Ptgds,* and *Vars2,* encoding enzymes; *Dagk4,* mouse homologue of *PSK, Nme2, Snf1lk* and *Tyki,* encoding kinases; *Dusp10*, *Inpp4a, Inpp5b, Pps, Ptpn1,* and *Ptpn5,* encoding phosphatases; *I116, Prg,* and *Scya4,* encoding secreted factors; *Akap7, Api5, Arfrp1, Arhgapl4-pending, Cish2, Dapp1, Fabp6, Fkbp8, Fliz1-pending, Hint, Ier5, Jundp2-pending, Lmo6, Mid1,* mouse homologue of *AKAP13,* mouse homologue of *BIN2,* mouse homologue of *CEZANNE,* mouse homologue of *CHD2,* mouse homologue of *MBLL,* mouse homologue of *SLC16A10,* mouse homologue of *SLC16A6,* mouse homologue of *SLC17A5,* mouse homologue of *TAF5L,* mouse homologue of *U1SNRNPBP,* mouse homologue of *ZNF8, Mtap7, Myolc, Nkx2-3, Nsf, Pcdh9, Pkig, Prdx2, Pscd1, Psmb1, Psme1, Psme2, Rg11*, *Ril-pending, Sax1 Slc14a2, Slc7a1, Slc7a11*, *Swap70, Txnip,* and *Ub13,* encoding signaling proteins; *Clic3, Gtl1-13,* mouse homologue of *NOL5A,* and *Vdac2,* encoding structural proteins; *ABT1-pending,* Ctbpl, Dermol, *Ebf, Elf4, Ldb1*, mouse homologue of *NR1D1*, mouse homologue of *ZER6, Rest, Tbp, Yy1, Zfp238, Zfp287,* and *Zfp319,* encoding proteins involved in transcriptional regulation; *Lrrc2*, *Satb1 Slfn4,* genes with the following Celera identification codes mCG10290, mCG10613, mCG11234, mCG11325, mCG11355, mCG11803, mCG11817, mCG12566, mCG12630, mCG12824, mCG13346, mCG14143, mCG14155, mCG14342, mCG15141, mCG15321, mCG16761, mCG16858, mCG17127, mCG17140, mCG17142, mCG17547, mCG17569, mCG17751, mCG17799, mCG17802, mCG17918, mCG18034, mCG1850, mCG18663, mCG18737, mCG20276, mCG20905, mCG20994, mCG21403, mCG21505, mCG21529, mCG21530, mCG21803, mCG22014, mCG22045, mCG22386, mCG2258, mCG22772, mCG23032, mCG23035, mCG23069, mCG23075, mCG23120, mCG2543, mCG2824, mCG2947, mCG3038, mCG3729, mCG3760, mCG50409, mCG50651, mCG5068, mCG5070, mCG51393, mCG52252, mCG52498, mCG53009, mCG53724, mCG55023, mCG55075, mCG55198, mCG55265, mCG55512, mCG56069, mCG56089, mCG56746, mCG57132, mCG57265, mCG57617, mCG57827, mCG58254, mCG58345, mCG5900, mCG5905, mCG59368, mCG59375, mCG59533, mCG59662, mCG59810, mCG59997, mCG60526, mCG60833, mCG61221, mCG61661, mCG61897, mCG61907, mCG61943, mCG62177, mCG62971, mCG63537, mCG63601, mCG64346, mCG64382, mCG64398, mCG65022, mCG65585, mCG65785, mCG66128, mCG66379, mCG66776, mCG66965, mCG7831, mCG7856, mCG8424, mCG9002, mCG9537, mCG9538, mCG9791, mCG9792, mCG9843, mCG9875, mCG9877, and mCG9880. These genes are also listed in Table 1.

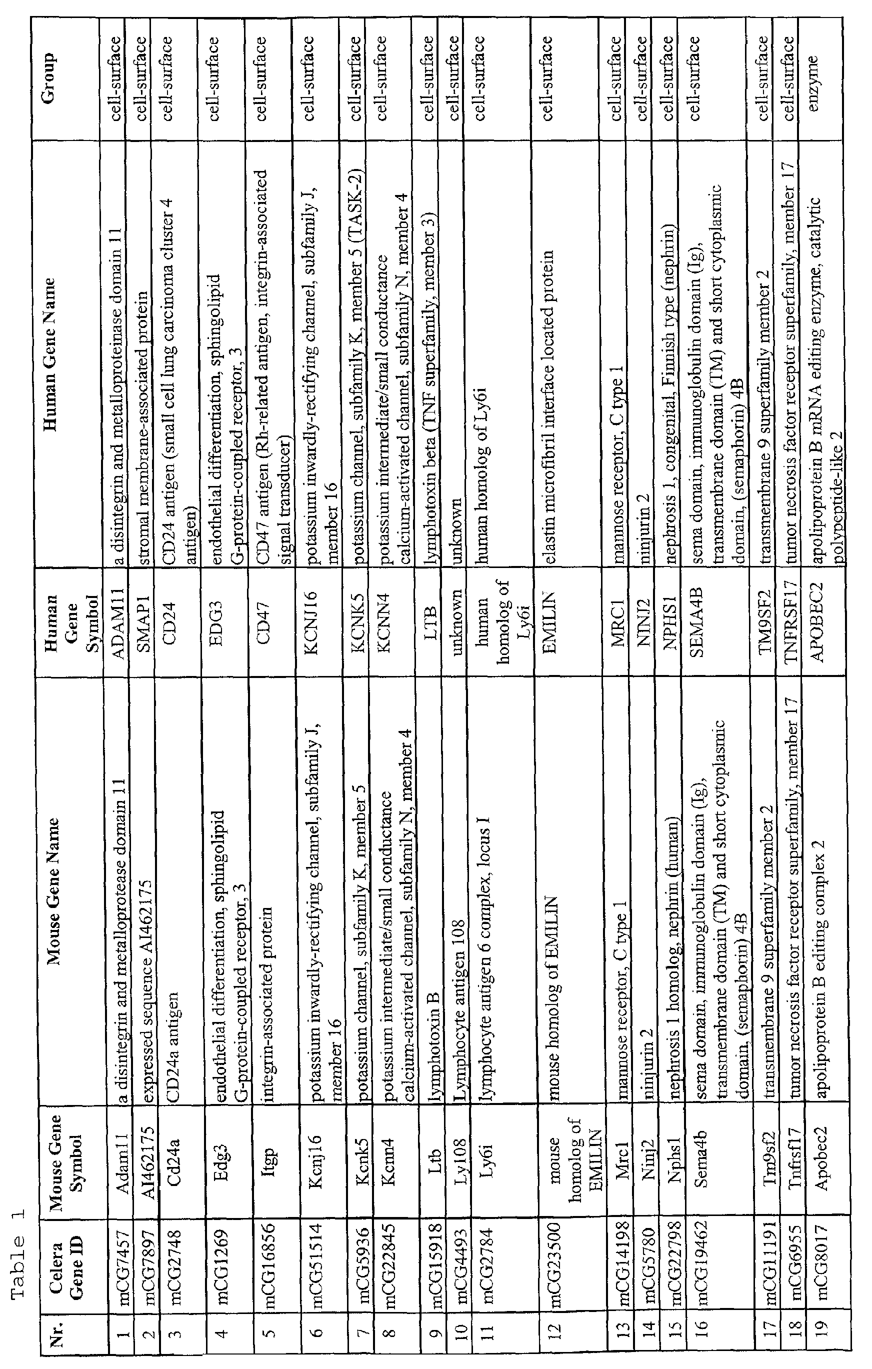

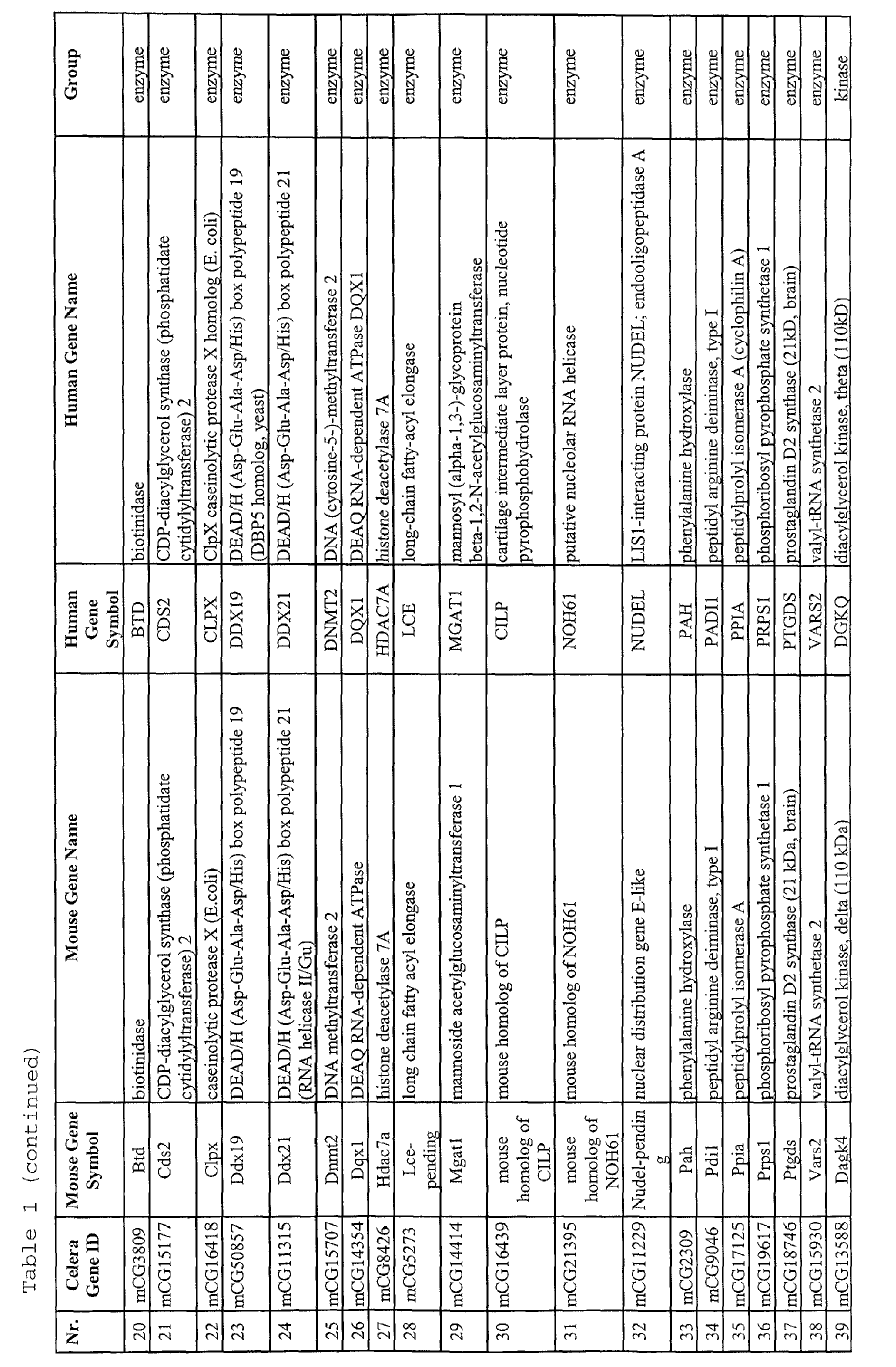

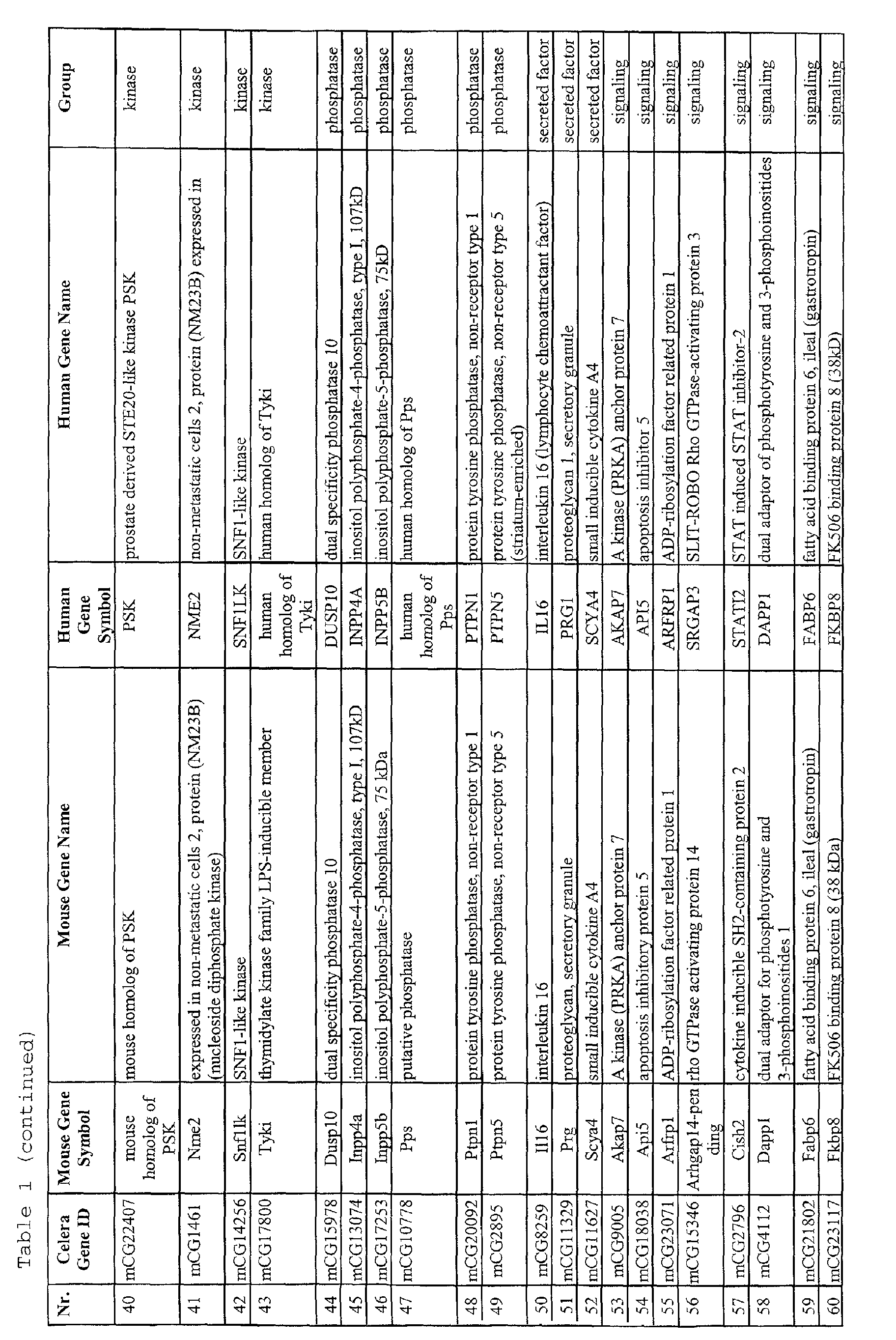

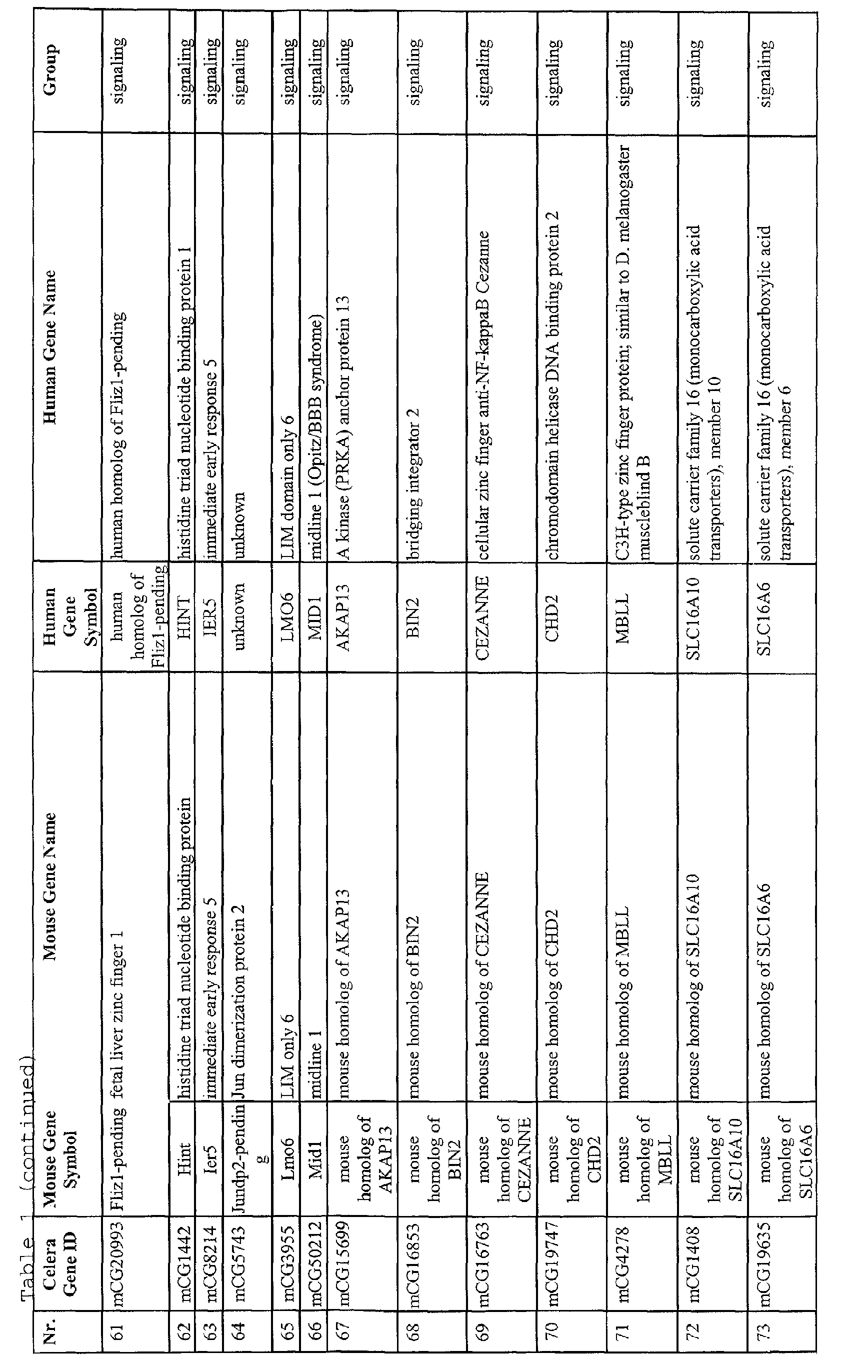

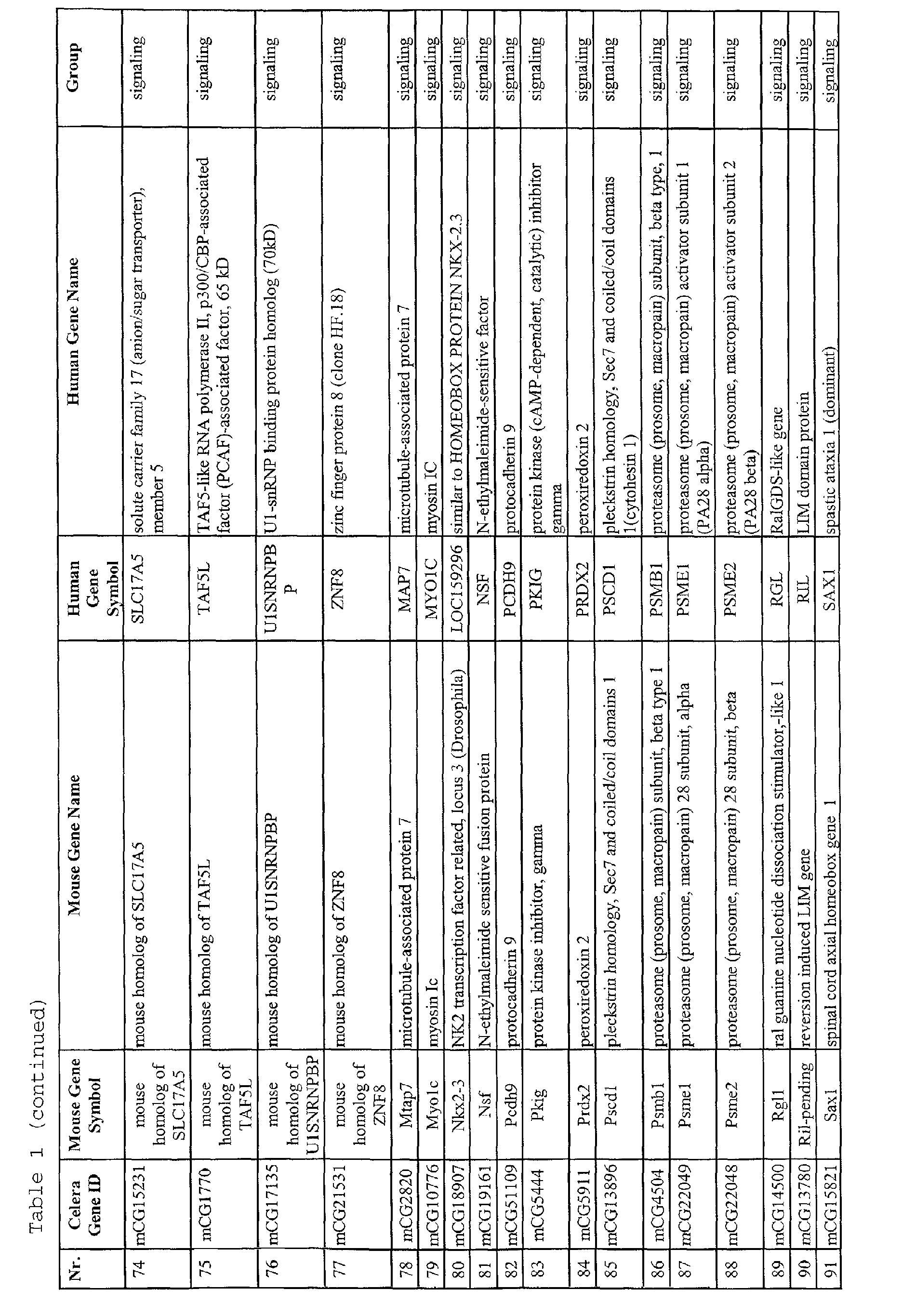

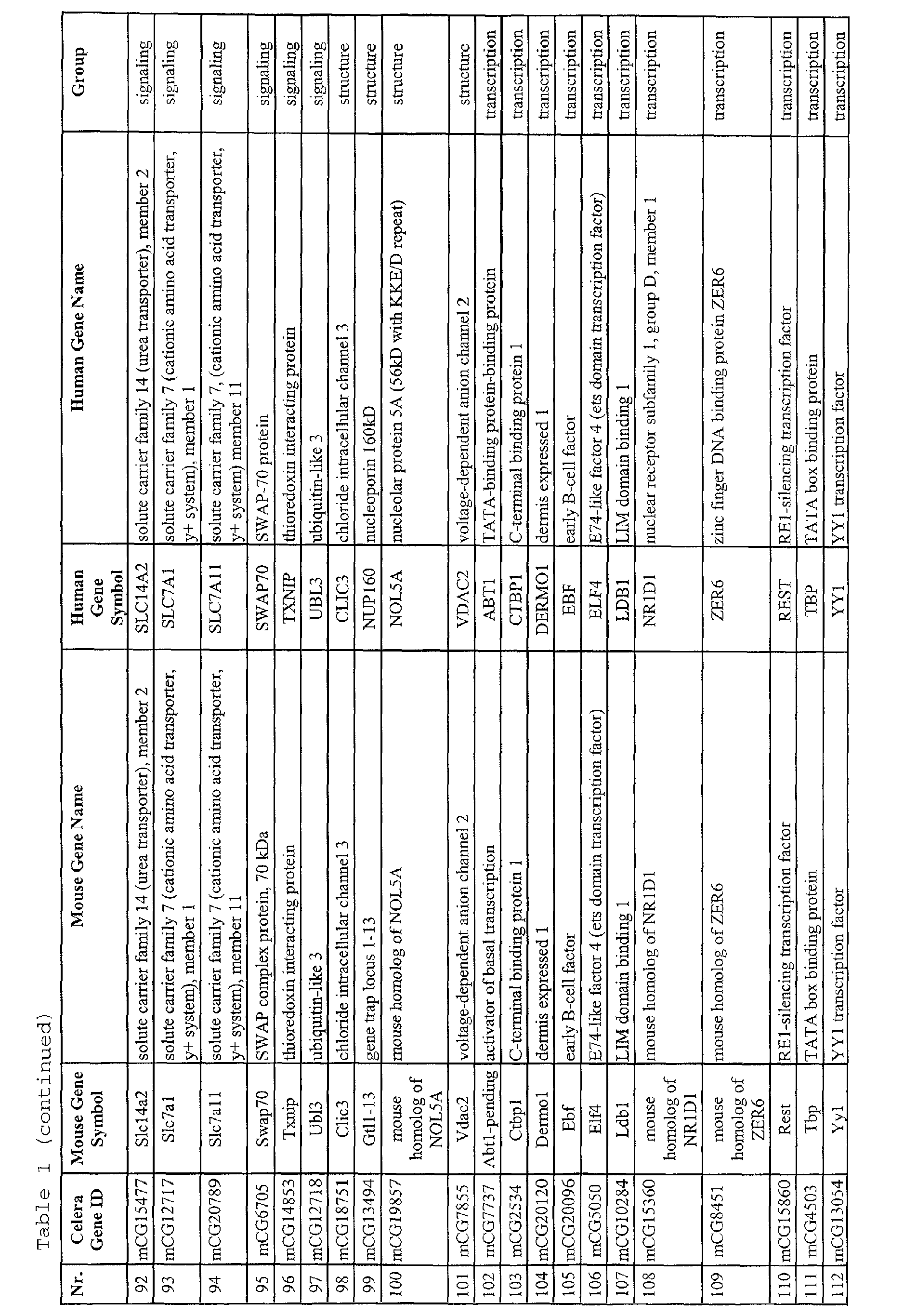

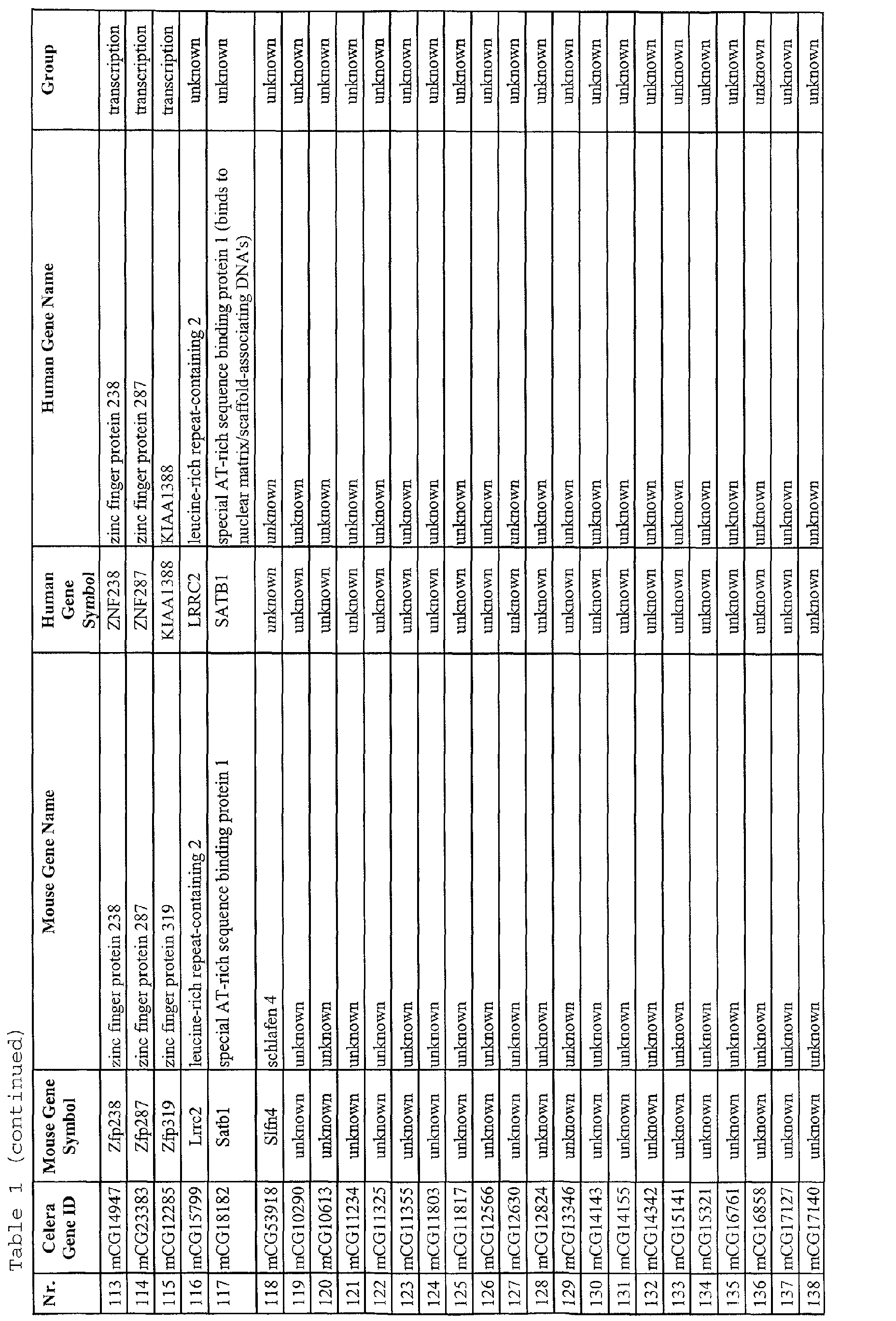

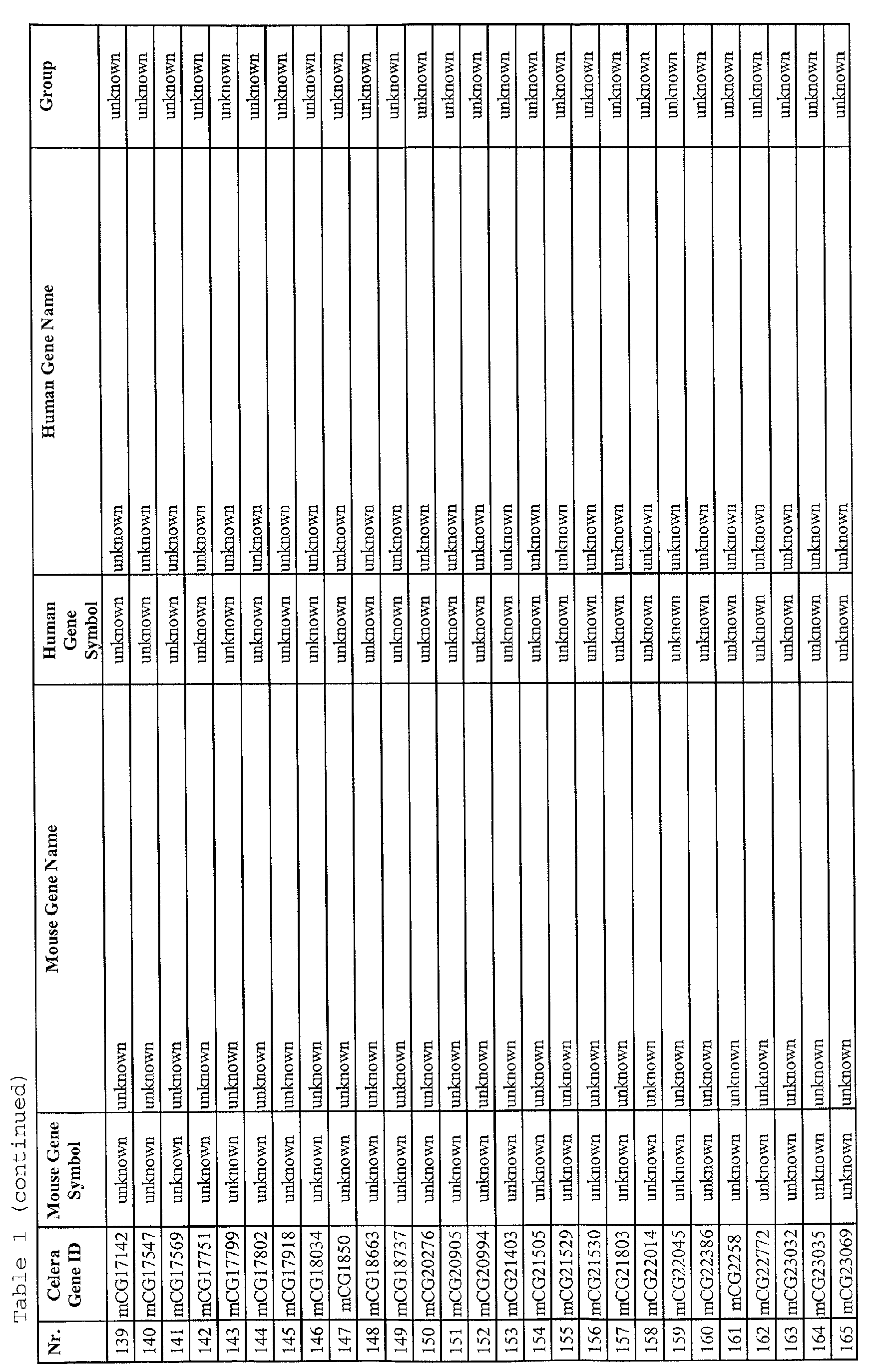

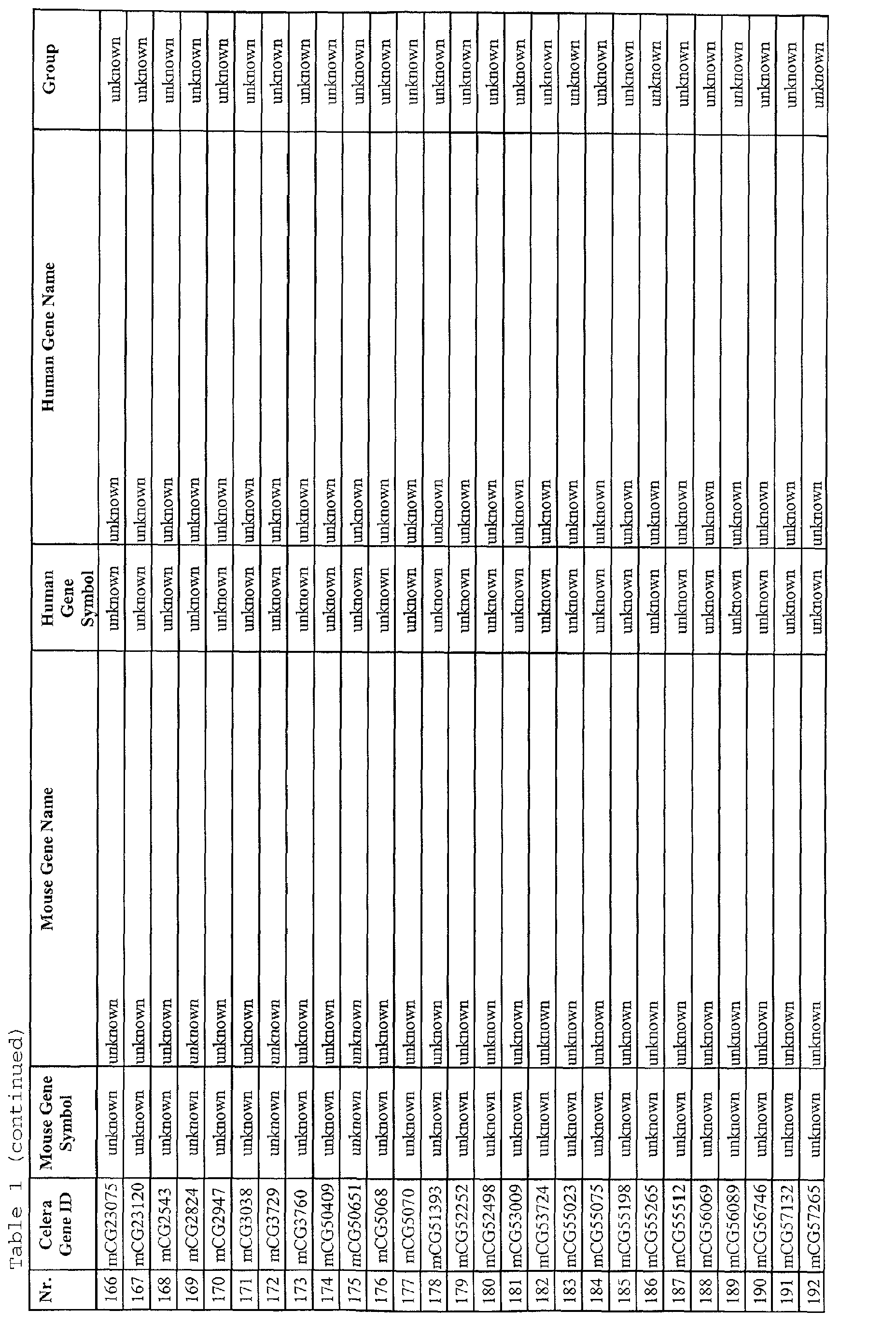

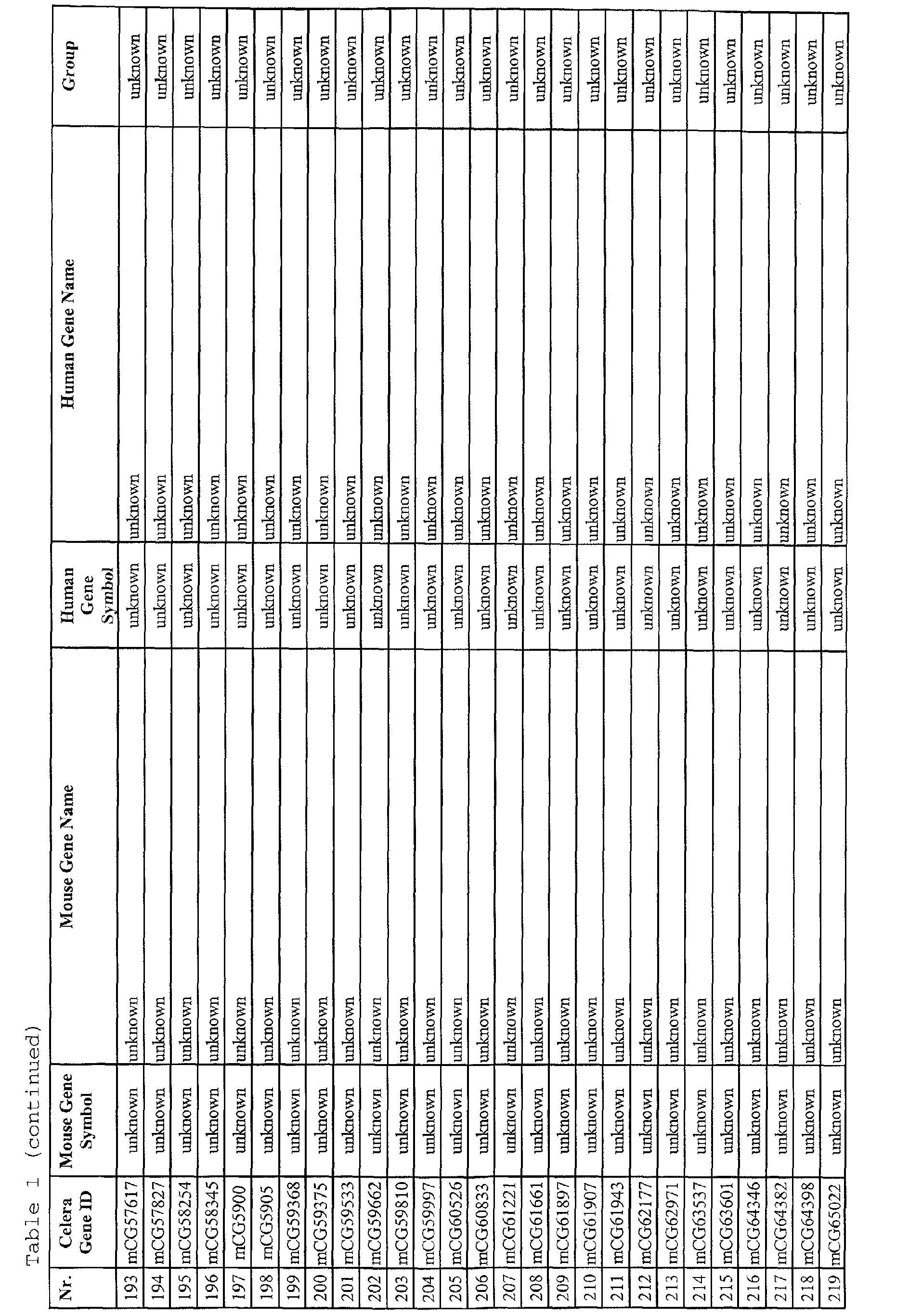

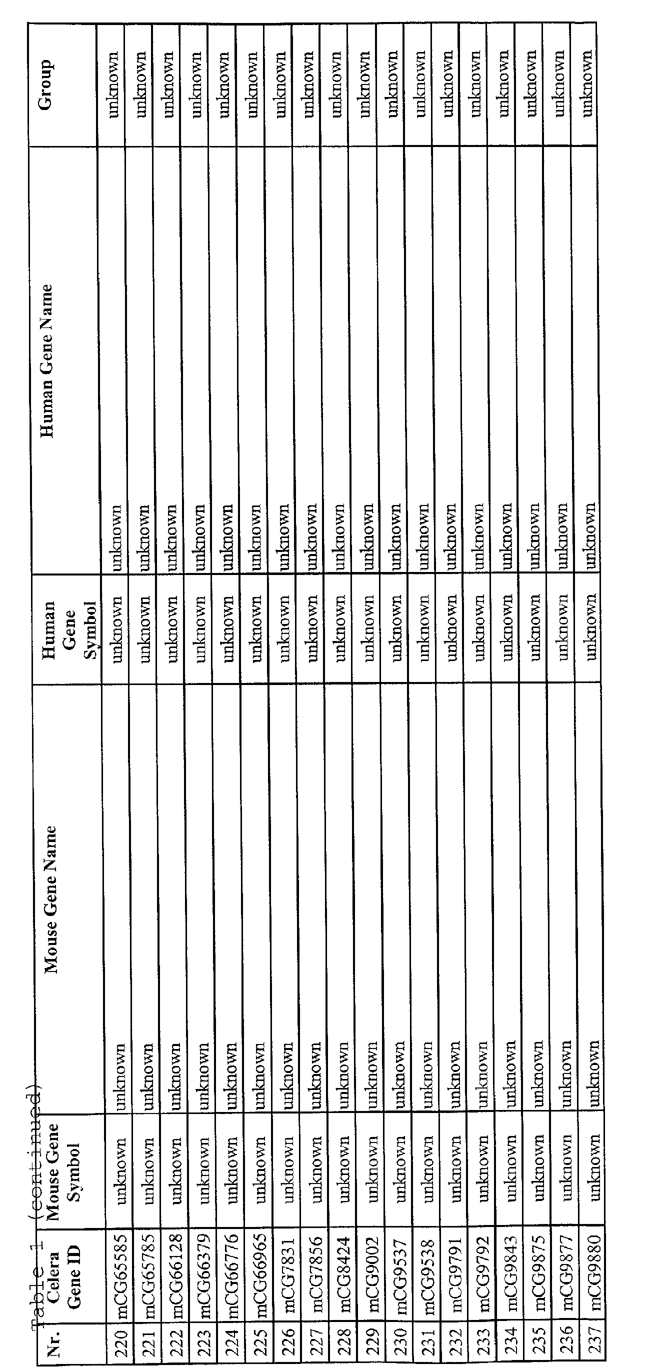

The first object of the present invention to provide novel genes involved in tumor development for use in the design of tumor specific therapies is thus achieved by using the human homologues of the murine genes of Table 1 to develop inhibitors directed against these genes and/or their expression products and to use these inhibitors for the preparation of pharmaceutical compositions for the treatment of cancer.

The term "human homologue" as used herein should be interpreted as a human gene having the same function as the gene identified in mouse.

In one embodiment of the present invention, the inhibitors are antibodies and/or antibody derivatives directed against the expression products of the genes listed in Table 1. Such antibodies and/or antibody derivatives such as scFv, Fab, chimeric, bifunctional and other antibody-derived molecules can be obtained using standard techniques generally known to the person skilled in the art. Therapeutic antibodies are useful against gene expression products located on the cellular membrane. Antibodies may influence the function of their target proteins by for example steric hindrance or blocking at least one of the functional domains of those proteins. In addition, antibodies may be used for deliverance of at least one toxic compound linked thereto to a tumor cell.

In a second embodiment of the present invention, the inhibitor is a small molecule capable of interfering with the function of the protein encoded by the gene involved in tumor development. In addition, small molecules can be used for deliverance of at least one linked toxic compound to a tumor cell.

Small molecule inhibitors are usually chemical entities that can be obtained by screening of already existing libraries of compounds or by designing compounds based on the structure of the protein encoded by a gene involved in tumor development. Briefly, the structure of at least a fragment of the protein is determined by either Nuclear Magnetic Resonance or X-ray crystallography. Based on this structure, a virtual screening of compounds is performed. The selected compounds are synthesized using medicinal and/or combinatorial chemistry and thereafter analyzed for their inhibitory effect on the protein in vitro and in vivo. This step can be repeated until a compound is selected with the desired inhibitory effect. After optimization of the compound, its toxicity profile and efficacy as cancer therapeutic is tested in vivo using appropriate animal model systems.

The expression level of a gene can either be decreased or increased during tumor development. Naturally, inhibitors are used when the expression levels are elevated.

On a different level of inhibition nucleic acids can be used to block the production of proteins by destroying the mRNA transcribed from the gene. This can be achieved by antisense drugs or by RNA interference (RNAi). By acting at this early stage in the disease process, these drugs prevent the production of a disease-causing protein. The present invention relates to antisense drugs, such as antisense RNA and antisense oligodeoxynucleotides, directed against the genes listed in Table 1. Each antisense drug binds to a specific sequence of nucleotides in its mRNA target to inhibit production of the protein encoded by the target mRNA. The invention furthermore relates to RNAi molecules. RNAi refers to the introduction of homologous double stranded RNA to specifically target the transcription product of a gene, resulting in a null or hypomorphic phenotype. RNA interference requires an initiation step and an effector step. In the first step, input double-stranded (ds) RNA is processed into 21-23-nucleotide 'guide sequences'. These may be single- or double-stranded. The guide RNAs are incorporated into a nuclease complex, called the RNA-induced silencing complex (RISC), which acts in the second effector step to destroy mRNAs that are recognized by the guide RNAs through base-pairing interactions. RNAi molecules are thus double stranded RNAs (dsRNAs) that are very potent in silencing the expression of the target gene. The invention provides dsRNAs complementary to the genes listed in Table 1.

The invention relates further to gene therapy, in which the genes listed in Table 1 are used for the design of dominant-negative forms of these genes which inhibit the function of their wild-type counterparts following their directed expression in a cancer cell.

A further aspect of the present invention relates to the use of the murine genes as well as their human homologues listed in Table 1 or their products for the development of reagents for diagnosis of cancers.

The invention also provides diagnostic compositions for diagnosing cancer, comprising histological examination of tissues specimens using specific antibodies directed against the products of the genes listed in Table 1 and/or in-situ hybridisation analysis of gene expression using specific RNA probes directed these genes.

Another object of the present invention is to provide a pharmaceutical composition comprising the inhibitors according to the present invention as active ingredient for the treatment of cancer. The composition can further comprise at least one pharmaceutical acceptable additive like for example a carrier, an emulsifier, or a conservative.

In addition, it is the object of the present invention to provide a method for treatment of cancer patients which method comprises the administration of the pharmaceutical composition according to the invention to cancer patients.

The invention will be further illustrated in the examples that follow and which are not given to limit the invention. Example 1 describes how the genes of Table 1 were identified. Example 2 describes the analysis of the YY1 gene with respect to its involvement in tumor development. Example 3 describes the development of inhibitors of the genes and their encoded products.

### Examples

### EXAMPLE 1

### Identification of common viral insertion sites (CIS)

### INTRODUCTION

To identify common viral integration sites in mouse tumors and cell lines, the MuLV virus was used. Mice and cell lines were either infected with murine leukemia virus 1.4 (Graffi-1.4 MuLV) or with Cas-Br-M MuLV.

The Graffi-MuLV is an ecotropic retroviral complex causing leukemias in mice. This viral complex does not contain oncogenic sequences itself but rather deregulates genes due to proviral integrations. Graffi-1.4 MuLV is a subclone of this complex and predominantly induces myeloid leukemias.

NIH/Swiss mice infected with Cas-Br-M MuLV develop myeloid or lymphoid malignancies also as a result of retroviral insertion that affect target genes.

### MATERIALS AND METHODS

### 1. Induction of leukemias

Newborn FVB/N mice or NIH/Swiss were injected subcutaneously with 100 µl of a cell culture supernatant of Graffi-1.4 MuLV or Cas-Br-M MuLV producing NIH3T3 cells, respectively. Mice were checked daily for symptoms of illness, i.e., apathy, white ears and tail, impaired interaction with cage-mates, weight-loss, and dull fur. Typically, leukemic mice suffered from enlarged spleens, livers, thymuses, and lymph nodes. From these primary tumors, chromosomal DNA was isolated for PCR-based screening. Blood samples were taken from the heart. For morphological analysis, blood smears and cytospins were fixed in methanol, May-Grunwald-Giemsa (MGG) stained and analyzed.

Single-cell suspensions of different organs were analyzed by flow cytometry using a flow cytometer. The cells were labeled with the following rat monoclonal antibodies: ER-MP54 (ER-MP54), ER-MP58 (ER-MP58), M1/70 (Mac-1), F4/80 (F4/80), RB68C5 (GR-1), ER-MP21 (transferrin receptor), TER119 (Glycophorin A), 59-AD2.2 (Thy-1), KT3 (CD3), RA3 6B2 (B220) and E13 161-7(Seal). Immunodetection was performed utilizing a Goat-anti-Rat antibody coupled to fluorescein isothiocyanate.

### 2. Inverse PCR on Graffi-1.4 MuLV induced leukemias

Genomic DNA from the primary tumors was digested with HhaI. After ligation, a first PCR was performed using Graffi-1.4 MuLV (LTR) specific primers L1 (5' TGCAAGATGGCGTTACTGTAGCTAG 3') and L2 (5' CCAGGTTGCCCCAAAGACCTG 3') (cycling conditions were 1 min at 94°C, 1 min at 65°C, 3 min at 72°C [30 cycles]). For the second nested PCR the primers L1N (5' AGCCTTATGGTGGGGTCTTTC 3') and L2N (5' AAAGACCTGAAACGACCTTGC 3') (15 cycles) were used. The PCR reaction mixture contained 10 mM Tris-HCl (pH 8.3), 50 mM KCL, 1.5 mM MgCl₂, 200 µM dNTP's, 10 pmol of each primer and 2.5 units of Taq-polymerase. The PCR fragments were analyzed on a 1% agarose gel and cloned in a TA cloning vector according to standard procedures.

PCR products were sequenced with the M13 forward primer 5' GACCGGCAGCAAAATG 3' and M13 reverse primer 5' CAGGAAACAGCTATGAC 3'. Virus flanking genomic sequences were identified using the National Center for Biotechnology Information (NCBI) and Celera databases.

### 3. Inverse PCR and RT-PCR on Cas-Br-M MuLV leukemias

Five µg of genomic DNA was digested with *Sau3A* or with *SstI.* The products were treated with T4-ligase, which resulted in the formation of circularized products. Subsequently, an inverse PCR (ICPR) strategy was used with primers specific for the Cas-Br-M MuLV LTR. For the *Sau3A* digested/ligated fragments, the first PCR reaction was carried out with primers pLTR4 (5'-CCG AAA CTG CTT ACC ACA-3') and pLTR3 (5'-GGT CTC CTC AGA GTG ATT-3'), followed by a nested PCR using pLTR5 (5'-ACC ACA GAT ATC CTG TTT-3') and pLTR6 (5'-GTG ATT GAC TAC CCG CTT-3'). Cycle conditions for both PCRs were 15" at 94°C, 30" at 57°C, and 2' at 72°C for 10 cycles, and an additional 20 cycles following the conditions 15" at 94°C, 30" at 57°C, and 2'30" at 72°C. Reactions were performed using Expand High Fidelity PCR System. In case of *SstI* digested genomic DNA, the circularized DNA was amplified using primers pLTR9 (5'-GAC TCA GTC TAT CGG AGG AC-3') and pLTR1 (5'-CTT GCT GTT GCA TCC GAC TGG-3'), and pLTR10 (5'-GTG AGG GGT TGT GTG CTC-3') and 2 (5'-GTC TCG CTG TTC CTT GGG AGG-3'), respectively. The first PCR was performed for 30 cycles 30" at 94°C, 1' at 60°C, and 3' at 72°C. The reaction was carried out with Expand High Fidelity PCR system. Nested PCR conditions were 30 cycles of 30" at 94°C, 1' at 58°C, and 1' at 72°C. This reaction was performed with Taq polymerase.

For RT-PCR, total RNA was isolated through a CsCl gradient. First strand cDNA was obtained by reverse transcriptase (RT) reactions with an oligo(dT)-adapter primer (5'-GTC GCG AAT TCG TCG ACG CG(dT)₁₅-3') at 37°C with 5 µg RNA, using the Superscript™ Preamplification System. Subsequently, PCRs (1' at 94°C, 1' at 58°C, 3' at 72°C (30 cycles)) were performed on the RT reactions of the leukemias by using the LTR specific primer pLTR6 and the adapter primer (5'-GTC GCG AAT TCG TCG ACG CG-3'). PCR products were directly cloned into pCR2.1 and subjected to nucleotide sequencing with the M13 forward primer 5'GACCGGCAGCAAAATG 3' and m13 reverse primer 5'CAGGAAACAGCTATGAC 3'. Nucleotide sequences were compared to the NCBI and Celera databases for analysis.

### Results

### 1. Graffi-1.4 MuLV induced leukemias

Leukemias developed 4 to 6 months after subcutaneous injection of newborn FVB/N mice with Graffi-1.4 MuLV. Forty-eight of the 59 leukemias (81%) analyzed exhibited morphological characteristics of myeloid cells. Blast cell percentages in the bone marrow ranged from 24 to 90% with an average of 48%. Leukemia cells expressed immunophenotypic marker profiles consistent with their myeloid appearance, e.g., ER-MP54+, ER-MP58+, CD3-, GR-1+. Six leukemias with blast-like morphology showed no immunophenotypic differentiation markers, suggesting that these tumors represented very immature leukemias. Only 3 leukemias were of T-lymphoid origin (CD3+/MP58-/Thy1+) and 2 showed mixed myeloid and erythroid features (Terll9+/ER-MP58+/F4/80+). These results demonstrate that Graffi-1.4 MuLV infection predominantly induce myeloid leukemia in FVB/N mice.

The provirus flanks were cloned, subjected to nucleotide sequencing, and blasted against the Celera and NCBI databases resulting in the identification of common insertion sites. Of the genes identified, the ones that were so far not described to be involved in tumor development are listed in Table 1 combined with the novel cancer genes identified from Cas-Br-M MuLV induced leukemias.

### 2. Cas-Br-M MuLV induced leukemias

Cas-Br-M MuLV injected newborn NIH/Swiss mice developed leukemias by approximately 140 to 400 days postinoculation. Most of these were myeloid leukemias (59%), although T-cell (21%), and mixed T-cell/myeloid (21%) leukemias were found.

To clone viral integration sites, a virus-LTR (long terminal repeat) specific inverse PCR as well as RT-PCR were applied as complementary approaches using DNA or RNA from 35 myeloid leukemias. The inverse PCR method was carried out on 19 primary leukemias and 9 cell lines, whereas the RT-PCR based technique was performed on 12 cell lines and 2 primary leukemias.

The provirus flanks were subjected to nucleotide sequencing, blasted against the Celera and NCBI databases resulting in the identification of common insertion sites. Of the genes identified, the ones that were so far not described to be involved in tumor development are listed in Table 1 combined with the novel cancer genes identified from Graffi-1.4 MuLV induced leukemias.

### DISCUSSION

Although proviral integrations occur randomly, they may affect the expression or function of nearby genes. If a gene is affected in two or more independent tumors, this indicates that these integrations provide a selective advantage and therefore contribute to tumor development. Multiple of these common insertion sites were identified of which a large number are demonstrated for the first time to play a role in cancer. Importantly, several of the other genes identified are well-known cancer genes validating the approach. This example shows that the pursued strategy can be successfully used to identify novel genes that are involved in tumor development.

### EXAMPLE 2

### Analysis of proviral integration sites in the YY1 gene and their effect on tumorigenisis

### INTRODUCTION

YY1 is a transcription factor of the CLI-Krüppel zinc finger protein family that has been reported to activate or repress transcription of a large variety of cellular and viral genes. Additionally, YY1 regulates gene expression in a cell-cycle dependent fashion. This may at least in part be due to a control mechanism involving the retinoblastoma protein (Rb), which releases YY1 in the S-phase of the cell cycle. The transcriptional activity of YY1 is positively regulated through acetylation of the protein by p300 and PCAF and negatively regulated by deacetylation by histone deacetylases HDAC1, HDAC2 and HDAC3.

This example describes that the *YY1* gene is commonly targeted in Graffi-1.4 MuLV-induced leukemias and that deregulation of *YY1* expression leads to defects in myeloid cell development and contributes to leukemogenesis.

### MATERIALS AND METHODS

### 1. Graffi-1.4 MuLV -induced leukemias

Newborn FVB/N mice were injected subcutaneously with 100 µl of a cell culture supernatant of Graffi-1.4 MuLV - producing NIH3T3 cells. Mice were checked daily for symptoms of illness, i.e., apathy, white ears and tail, impaired interaction with cage-mates, weight-loss, and dull fur. Typically, leukemic mice suffered from enlarged spleens, livers, thymuses, and lymph nodes. From these primary tumors, chromosomal DNA was isolated for PCR-based screening. Blood samples were taken from the heart. For morphological analysis, blood smears and cytospins were fixed in methanol, May-Grunwald-Giemsa (MGG) stained and analyzed.

### 2. Immunophenotyping of the leukemic cells

Single-cell suspensions of different organs were analyzed by flow cytometry using a flow cytometer. The cells were labeled with the following rat monoclonal antibodies: ER-MP54 (ER-MP54), ER-MP58 (ER-MP58), M1/70 (Mac-1), F4/80 (F4/80), RB68C5 (GR-1), ER-MP21 (transferrin receptor), TER119 (Glycophorin A), 59-AD2.2 (Thy-1), KT3 (CD3), RA3 6B2 (B220) and E13 161-7(Sca1). Immunodetection was performed utilizing a Goat-anti-Rat antibody coupled to fluorescein isothiocyanate.

### 3. Production of retroviral vectors

The plasmid pCMV-HA-YY1, containing hemagglutinin (HA)-tagged full length human YY1 cDNA, was digested with XbaI and ApaI, and the HA-YY1 fragment was blunted and ligated into the HpaI site of pLNCX and pBabe retroviral vectors. PhoenixA packaging cells were transfected with pLNCX-HA-YY1 or pBABE-HA-YY1. Supernatants containing high-titer, helper-free recombinant virus were harvested from 80% confluent producer cells cultured for 16-20 hours in Dulbecco's modified Eagle's medium supplemented with 5% fetal calf serum (FCS), penicillin (100 IU/ml) and streptomycin 100 ng/ml). To determine titers of BABE-HA-YY1 and BABE control virus, the virus particles were pelleted by ultracentrifugation at 41,000 rpm and RNA was extracted with phenol (pH=4.0) and spot-blotted on nitrocellulose filters. This blot was hybridized with a BABE specific probe (SV40 fragment, BamHI-HindIII digest).

### 4. Cell culture and retroviral gene transfer

The interleukin-3 (IL-3) dependent murine myeloid cell line 32D containing the human wild type G-CSF- receptor (32D-WT1) was infected with pLNCX-HA-YY1 virus and selected with G418. Several independent clones were expanded for further analysis.

Hematopoietic cells were harvested from the femurs and tibiae of 8 to 12 week old FVB mice. After depletion of adherent cells, the remaining cells were fractionated on a Percoll™ gradient. Fraction I (density 1.058/1.0645) containing the earliest hematopoietic progenitor cells was collected. Cells were washed twice in HBSS/5% FCS/0.5% BSA and then prestimulated for 2 days at a final concentration of 5x10⁵ cells/ml in Cell Gro® supplemented with a cytokine cocktail composed of mIL-3 (10 ng/ml), hFlt3-ligand, hTPO, mSCF (100 ng/ml) and GM-CSF (2 u/ml). Retroviral infection was performed in culture dishes coated with the recombinant fibronectin fragment CH-296 at a concentration of 12 mg/ml. Before adding the bone marrow cells, the dishes were preincubated with virus supernatant (BABE-HA-YY1 or empty BABE) for 30 min at 37°C. Subsequently, bone marrow cells were resuspended and mixed with fresh virus supernatant in a 1:1 ratio and a fresh cytokine cocktail was added. The cells (5x10⁵) were cultured overnight at 37°C and 5% CO₂. Virus supernatant and cytokine cocktail were refreshed again the next day and cells were cultured for another 24 hrs.

For colony assays, bone marrow cells were plated at densities of 1 to 5x10⁴ cells per ml per dish in triplicate in methylcellulose medium supplemented with 30% FBS, 1% BSA, 0.1mM 2-mercaptoethanol, 2mM L-glutamine, GM-CSF (20 u/ml) and with or without 2.5 mg/ml puromycin to evaluate the infection efficiency of the different retroviruses. Colonies consisting of more than 50 cells were counted on day 7 of culture.

### 5. Promoter activity assay

The YY1 promoter containing subclone pdSS4.5 of 124 was digested with restriction enzymes MluI and BglII. This YY1 promoter fragment was cloned in the luciferase reporter plasmid pGL3. The Graffi-1.4 MuLV LTR sequence was cloned in both orientations in the HpaI site. The Sp1 site was mutated with the QuikChange Site-Directed Mutagenesis Kit by using primer SPIMUTF: 5' GGACGGTTCGGGGCGAGAGC 3' and primer SPIMUTR: 5' GCTCTCGCCCCGAACCGTCC 3'. As a positive control pRSV-Luc was used. The pRSV-β-galactosidase was used for reference. Empty pGL3 vector served as a negative control. Luciferase assays were performed in HEK 293 cells according to standard procedures. Cells were transfected by CaPO₄ precititation with a mixture of 5 mg derived pGL3 plasmid containing different promoter sequences and 2.5 mg pRSV-β-galactosidase, supplemented with empty vector to a total amount of 20 mg of total plasmid per ml.

### 6. Inverse PCR on Graffi-1.4 MuLV induced leukemias

Genomic DNA from the primary tumors was digested with HhaI. After ligation, a first PCR was performed using Graffi-1.4 MuLV (LTR) specific primers L1 (5' TGCAAGATGGCGTTACTGTAGCTAG 3') and L2 (5' CCAGGTTGCCCCAAAGACCTG 3') (cycling conditions were 1 min at 94°C, 1 min at 65°C, 3 min at 72°C [30 cycles]). For the second nested PCR the primers L1N (5' AGCCTTATGGTGGGGTCTTTC 3') and L2N (5' AAAGACCTGAAACGACCTTGC 3') (15 cycles) were used. The PCR reaction mixture contained 10 mM Tris-HCl (pH 8.3), 50 mM KCL, 1.5 mM MgCl₂, 200 µM dNTP's, 10 pmol of each primer and 2.5 units of Taq-polymerase. The PCR fragments were analyzed on a 1% agarose gel and cloned in the TA cloning vector according to standard procedures.

### 7. Detection of virus integration in the YY1 gene by specific nested PCRs

To determine the orientation and localization of the Graffi-1.4 provirus in the YY1 gene in an extended panel of leukemias, a nested PCR was performed on 1 mg of genomic DNA from primary tumors. For the first PCR, YY1 promoter region specific primers Y1 5' AGGAATCAGGAGCAGAAGAAAGTTTTGGGA 3' and Y2 5' CAATAAAGTCTGCTCTGACGAGAAACGCC 3' in combination with Graffi-1.4 MuLV LTR specific primers L1 and L2 were used. Cycling conditions were 1 min 94°C, 1 min 65 °C, 3 min 72°C, 30 cycles. For the second PCR, the nested primers Y1N 5' AAACTCTCTGACTTACCTCCCTCTCCAAAGA 3' and Y2N 5' GTTCGTTTTGCCTTTACTCGTTACTCGGG 3' in combination with the nested primers L1N and L2N (30 cycles) were used. The obtained PCR products were analyzed by Southern blotting. To determine the orientation of the Graffi-1.4 provirus, the blots were hybridized with radiolabeled YIP 5' AAAACCTGCACAAGGACACCTTGCTAAGTATGTTT 3' and Y2P 5' AGCACACGGTCGGCTACGCTCCGTCCGCTACCGCA 3' at 45°C in Church buffer (0.5 M phosphate buffer, pH 7.2, 7% (w/v) SDS, 10 mM EDTA) overnight. Signals were visualized by autoradiography according to standard procedures.

### 8. Nucleotide sequencing

PCR products were cloned in the TA cloning vector and sequenced with the M13 forward primer 5' GACCGGCAGCAAAATG 3' and M13 reverse primer 5' CAGGAAACAGCTATGAC 3'. Virus flanking genomic sequences were identified using the National Center for Biotechnology Information (NCBI) and Celera databases.

### 9. Western blotting

Lysates of 32D cells were prepared and subjected to Western blotting. Antibodies used to visualize YY1 were Goat- anti-YY1 or rabbit anti-hemagglutinin (HA) for HA-tagged YY11. Goat-anti-Actin was used to control for equal loading of lysates.

### RESULTS

### 1. Graffi-1.4 MuLV-induced leukemias

Leukemias developed 4 to 6 months after subcutaneous injection of newborn FVB/N mice with Graffi-1.4 MuLV. Forty-eight of the 59 leukemias (81%) analyzed exhibited morphological characteristics of myeloid cells. Blast cell percentages in the bone marrow ranged from 24 to 90% with an average of 48%. Leukemia cells expressed immunophenotypic marker profiles consistent with their myeloid appearance, e.g., ER-MP54+, ER-MP58+, CD3-, GR-1+. Six leukemias with blast-like morphology showed no immunophenotypic differentiation markers, suggesting that these tumors represented very immature leukemias. Only 3 leukemias were of T-lymphoid origin (CD3+/MP58-/Thy1+) and 2 showed mixed myeloid and erythroid features (Ter119+/ER-MP58+/F4/80+). These results demonstrate that Graffi-1.4 MuLV infection predominantly induce myeloid leukemia in FVB/N mice.

### 2. Virus integrations in the YY1 gene

Inverse PCR was used to identify genomic sequences flanking Graffi-1.4 MuLV integrations. Integrations were found in several genes previously demonstrated to be involved in MuLV-induced leukemias, e.g., Notch-1, Nf1, p53, Fli-1 and Evi-1, as well as in novel loci. One of these newly identified integrations occurred in the YY1 locus, approximately 0.8 Kb upstream of the transcriptional initiation site. In a subsequent analysis on independent cell samples using nested PCR with LTR and YY1 primers it was found that 23 integrations were in the same region, i.e., 0.5 to 1.5 kb upstream of the transcriptional start site, in 14 out of 20 leukemias tested. Integrations occurred in both orientations in an approximately equal ratio. Searches for virus integrations in other parts of the gene using appropriate primer sets were negative.

### 3. Integration of Graffi-1.4 MuLV LTR deregulates YY1 transcription

Because integration of the virus occurred in both orientations, it is most likely that viral enhancer sequences in the LTR cause increased YY1 transcription in these leukemias. However, integration in the promoter region of a gene does not always result in alterations in expression. To study how virus integration influences YY1 expression, the Graffi-1.4 LTR was introduced at position -1417 in the YY1 promoter and transcriptional activity using a transient reporter assay was quantified. A two-fold higher YY1 promoter activity was measured following insertion of the Graffi-1.4 MuLV LTR, irrespective of the orientation of the LTR. Because a Sp1 binding site at position -48 to -39 has previously been shown to be important for induction of YY1 transcription, the effects of insertion of the Graffi-1.4 MuLV LTR after disruption of this site were also studied. While mutation of the Sp1 binding site resulted in a 50% reduction of normal promoter activity, the enhanced promoter activity caused by the integration of the LTR sequence was not affected. This result demonstrates that virus integration causes enhanced and Sp1-independent transcription of YY1.

### 4. Ectopic expression of YY1 blocks G-CSF-induced neutrophilic differentiation of 32D cells

The observation that integration of Graffi MuLV LTR alters the expression of YY1 in murine acute myeloid leukemia raised the question whether perturbed *YY1* expression might affect myeloid cell differentiation. 32D cell clones expressing human G-CSF receptors (32D-WT1 cells) were developed as a model to study neutrophilic differentiation.

Endogenous YY1 levels are high in parental 32D-WT1 cells cultured in IL-3 containing medium, under which conditions the cells remain immature. However, upon transfer of the cells to G-CSF-containing medium, in which cells undergo neutrophilic differentiation, YY1 protein levels declined from day 2 onward and remained low for the entire culture period. To determine the consequences of perturbed YY1 expression for myeloid cell development, we ectopically expressed HA-tagged *YY1* in these cells. As expected, *YY1* expression in 32D-HA-YY1 cells remained high after switching the cells to G-CSF. Growth rate and morphology of 32D-HA-YY1 cells cultured in IL-3 containing medium were similar to nontransduced control cells. Strikingly, when 32D-HA-YY1 cells were switched to G-CSF-containing medium, neutrophilic differentiation was markedly reduced. Instead, cells with a blast-like morphology expanded exponentially for 8 days in these cultures and rapidly died thereafter as a result of apoptosis. Thus, sustained *YY1* expression prevented G-CSF-induced myeloid differentiation of 32D cells without affecting the apoptotic response that normally accompanies G-CSF-induced differentiation.

### 5. Ectopic expression of YY1 blocks CFU-GM colony formation

The above-mentioned experiments show that constitutive expression of YY1 interferes with the G-CSF-induced myeloid differentiation in 32D cells. Next, it was investigated how perturbed expression of *YY1* affects the outgrowth of primary myelomonocytic progenitor cells (CFU-GM). To this end, mouse bone marrow cells were retrovirally transduced with BABE-HA-YY1 or empty BABE virus and plated in GM-CSF-containing colony assays. Notably, control bone marrow cells were subjected to equivalent titers of control virus to exclude that differences in colony outgrowth were caused by variations in transduction efficiencies. Strikingly, GM-CSF-induced colony formation by HA-YY1-transduced bone marrow cells was almost completely blocked. This result demonstrates that perturbed expression of *YY1,* instead of interfering with differentiation, causes a growth arrest or results in premature apoptosis of CFU-GM or their direct progeny.

### DISCUSSION

This example demonstrates that the gene encoding the transcriptional regulator YY1 is located in a common virus integration site in Graffi-1.4 MuLV-induced myeloid leukemia. The integrations occurred exclusively in the 5' promoter region of the gene, 0.5 to 1.5 kb upstream of the major transcriptional start site and resulted in a two-fold higher transcription of the YY1 gene.

Perturbed expression of YY1 inhibited G-CSF-induced myeloid differentiation in 32D cells and prevented the clonal outgrowth of CFU-GM progenitor cells. Importantly, it was observed that integration of Graffi-virus LTR sequences not only enhanced YY1 transcription, but also made it entirely independent of the recognition site for the transcription factor Sp1, which has been shown to play a pivotal role in YY1 expression. Although Sp1 expression has been reported to be high in hematopoietic cells, it is downregulated during differentiation in many cell types and may be involved in the decrease in YY1 protein levels observed during myeloid differentiation. Therefore, bypass of Sp1 transcriptional control might be one of the mechanisms by which viral integration deregulates YY1 protein levels in leukemic cells.

By demonstrating that perturbed expression of YY1 due to proviral integrations contributes to tumor development, this example validates the strategy of identifying novel cancer genes by retroviral insertional tagging.

### EXAMPLE 3

### Preparation of inhibitors of the expression products of genes involved in cancer from Examples 1 and 2

### Confirmation of the involvement of the identified genes in primary human tumors

The expression of the described genes, that were originally identified by genome-wide functional screens involving retroviral insertional tagging in mouse models (see Examples 1 and 2), is determined in a panel of different human tumor samples using microarray analysis. From an extensive set of primary human tumors of various organs, RNA is prepared using standard laboratory techniques to investigate the expression of the described genes in these samples relative to their expression in normal, unaffected tissues from the same origin by using microarrays on which these genes, or parts thereof, are spotted. Microarray analysis allows rapid screening of a large set of genes in a single experiment (DeRisi *et al.* Use of a cDNA microarray to analyse gene expression patterns in human cancer. Nat Genet 14:457-60, 1996; Lockhart et al. Expression monitoring by hybridization to high-density oligonucleotide arrays. Nat Biotechnol 14:1649, 1996). Genes that are differentially expressed in tumor and normal tissues as determined by microarray analysis are further examined by other techniques such as RT-PCR, Northern blot analysis, and, if gene-specific antibodies are available, Western blot analysis. Confirmation of differential expression of these genes demonstrates their involvement in human tumors. These findings are further substantiated by similar experiments using a panel of human tumor cell lines.

### Use of the identified genes as diagnostic tools for cancer

Genes that are differentially expressed in human tumors are used as diagnostic tools for cancer (according to Welsh *et al.* Analysis of gene expression profiles in normal and neoplastic ovarian tissue samples identifies candidate molecular markers of epithelial ovarian cancer. Proc Natl Acad Sci USA 98:1176-81, 2001). For example, certain genes are highly expressed in early-stage tumor cells compared with normal tissue. In a clinical setting, the expression of these genes is determined and used as a marker for the (pre-) malignant status of tissue samples according to Hegdeet al. (Identification of tumor markers in models of human colorectal cancer using a 19,200-element complementary DNA microarray. Cancer Res 61:7792-7, 2001).

### Functional importance of the identified genes for human cancer

Subsequently, the functional importance of the differentially expressed genes for tumor cells is determined by over-expression as well as by inhibition of the expression of these genes. Selected human tumor cell lines are transfected either with plasmids encoding cDNA of the genes or with plasmids encoding RNA interference probes for the genes. RNA interference is a recently developed technique that involves introduction of double-stranded oligonucleotides designed to block expression of a specific gene (see Elbashir *et al.* Duplexes of 21-nucleotide RNAs mediate RNA interference in cultured mammalian cells. Nature 411:494-8, 2001; Brummelkamp et al. A system for stable expression of short interfering RNAs in mammalian cells. Science 296:550-3, 2002). Using standard laboratory techniques and assays, the transfected cell lines are extensively checked for altered phenotypes that are relevant for the tumor cells, e.g. cell cycle status, proliferation, adhesion, apoptosis, invasive abilities, etc. These experiments demonstrate the functional importance of the identified genes for human cancer.

### Structure-function analysis of selected targets

Genes that are shown to be both differentially expressed and functionally important for human tumors are selected for structure-function analysis. Deletion and point mutation mutants of these genes are constructed and tested for their functional competence compared with wild-type genes (according to Ibanez et al. Structural and functional domains of the myb oncogene: requirements for nuclear transport, myeloid transformation, and colony formation. J Virol 62:1981-8, 1988; Rebay et al. Specific truncations of Drosophila Notch define dominant activated and dominant negative forms of the receptor. Cell 74:319-29, 1993). These studies lead to the identification of functionally critical domains as well as to dominant-negative variants, i.e. mutant genes that suppress the function of their endogenous counterparts (e.g. Kashles et al. A dominant negative mutation suppresses the function of normal epidermal growth factor receptors by heterodimerization. Mol Cell Biol 11:1454-63, 1991). These dominant-negatives provide additional proof for the functional importance of the genes and give insight into which therapeutic approaches can be pursued to interfere with their function. Furthermore, the cellular localization of the proteins encoded by the genes is determined by immunofluorescence using standard laboratory techniques. If the proteins are expressed on the cell-surface, this allows for the development of antibody-based inhibitors.

### Development of antibody-based inhibitors

Differentially expressed genes that encode membrane-bound proteins are selected as targets for conventional antibody-based therapies. Antibodies are generated against functionally relevant domains of the proteins and subsequently screened for their ability to interfere with the target's function using standard techniques and assays (Schwartzberg. Clinical experience with edrecolomoab: a monoclonal antibody therapy for colorectal carcinoma. Crit Rev Oncol Hematol 40:17-24, 2001; Herbst *et al.* Monoclonal antibodies to target epidermal growth factor receptor-positive tumors: a new paradigm for cancer therapy. Cancer 94:1593-611, 2002). These antibodies are also useful as diagnostic reagents (Syrigos *et al.* Use of monoclonal antibodies for the diagnosis and treatment of bladder cancer. Hybridoma 18:219-24, 1999).

### Development of small molecule inhibitors

Differentially expressed genes that do not encode membrane-bound proteins are selected as targets for the development of small molecule inhibitors. To identify putative binding sites or pockets for small molecules on the surface of the target proteins, the three-dimensional structures of those targets are determined by standard crystallization techniques (de Vos et al. Three-dimensional structure of an oncogene protein: catalytic domain of human c-H-ras p21. Science 239:888-93, 1988; Williamset al. Crystal structure of the BRCT repeat region from the breast cancer-associated protein BRCA1. Nat Struct Biol 8:838-42, 2001). Additional mutational analysis is performed as mentioned above to confirm the functional importance of the identified binding sites. Subsequently, Cerius2 (Molecular Simulations Inc., San Diego, CA, USA) and Ludi/ACD (Accelrys Inc., San Diego, CA, USA) software is used for virtual screening of small molecule libraries (Bohm. The computer program Ludi: A new method for the de novo design of enzyme inhibitors. J Comp Aided Molec Design 6:61-78, 1992). The compounds identified as potential binders by these programs are synthesized by combinatorial chemistry and screened for binding affinity to the targets as well as for their inhibitory capacities of the target protein's function by standard *in vitro* and *in vivo* assays. In addition to the rational development of novel small molecules, existing small molecule compound libraries are screened using these assays to generate lead compounds. Lead compounds identified are subsequently co-crystallized with the target to obtain information on how the binding of the small molecule can be improved (Zeslawska *et al.* Crystals of the urokinase type plasminogen activator variant beta(c)-uPAin complex with small molecule inhibitors open the way towards structure-based drug design. J Mol Biol 301:465-75, 2000). Based on these findings, novel compounds are designed, synthesized, tested, and co-crystallized. This optimization process is repeated for several rounds leading to the development of a high-affinity compound of the invention that successfully inhibits the function of its target protein. Finally, the toxicity of the compound is tested using standard assays (commercially available service via MDS Pharma Services, Montreal, Quebec, Canada) after which it is screened in an animal model system.

### Development of antisense molecule inhibitors

These inhibitors are either antisense RNA or antisense oligodeoxynucleotides (antisense ODNs) and are prepared synthetically or by means of recombinant DNA techniques. Both methods are well within the reach of the person skilled in the art. ODNs are smaller than complete antisense RNAs and have therefore the advantage that they can more easily enter the target cell. In order to avoid their digestion by DNAse, ODNs but also antisense RNAs are chemically modified. For targeting to the desired target cells, the molecules are linked to ligands of receptors found on the target cells or to antibodies directed against molecules on the surface of the target cells.

### Development of RNAi molecule inhibitors

Double-stranded RNA corresponding to a particular gene is a powerful suppressant of that gene. The ability of dsRNA to suppress the expression of a gene corresponding to its own sequence is also called post-transcriptional gene silencing or PTGS. The only RNA molecules normally found in the cytoplasm of a cell are molecules of single-stranded mRNA. If the cell finds molecules of double-stranded RNA, dsRNA, it uses an enzyme to cut them into fragments containing 21-25 base pairs (about 2 turns of a double helix). The two strands of each fragment then separate enough to expose the antisense strand so that it can bind to the complementary sense sequence on a molecule of mRNA. This triggers cutting the mRNA in that region thus destroying its ability to be translated into a polypeptide. Introducing dsRNA corresponding to a particular gene will knock out the cell's endogenous expression of that gene. This can be done in particular tissues at a chosen time. A possible disadvantage of simply introducing dsRNA fragments into a cell is that gene expression is only temporarily reduced. However, a more permanent solution is provided by introducing into the cells a DNA vector that can continuously synthesize a dsRNA corresponding to the gene to be suppressed. RNAi molecules are prepared by methods well known to the person skilled in the art.

### Efficacy of antibody-based and small molecule inhibitors in animal model systems

The efficacy of both the antibody-based and small molecule inhibitors are tested in an appropriate animal model system before entry of these inhibitors into clinical development (e.g. Brekken et al. Selective inhibition of vascular endothelial growth factor (VEGF) receptor 2 (KDR/Flk-1) activity by a monoclonal anti-VEGF antibody blocks tumor growth in mice. Cancer Res 60:5117-24, 2000; Wilkinson *et al.* Antibody targeting studies in a transgenic murine model of spontaneous colorectal tumors. Proc Natl Acad Sci USA 98:10256-60, 2001; Laird et al. SU6668 inhibits Flk-1/KDR and PDGFRbeta in vivo, resulting in rapid apoptosis of tumor vasculature and tumor regression in mice. FASEB J 16:681-90, 2002).

## Claims

1. Use of inhibitors of the expressed proteins of the murine genes and/or their human homologues listed in Table 1 for the preparation of a therapeutical composition for the treatment of cancer, in particular for the treatment of solid tumors of lung, colon, breast, prostate, ovarian, pancreas and leukemia.

2. Use as claimed in claim 1, wherein the inhibitors are antibodies or derivatives thereof directed against the expression products of the genes that are expressed on the cell membrane.

3. Use as claimed in claim 2, wherein the derivatives are selected from the group consisting of scFv fragments, Fab fragments, chimeric antibodies, bifunctional antibodies, and other antibody-derived molecules.

4. Use as claimed in claim 1, wherein the inhibitors are small molecules interfering with the biological activity of the protein expressed by the gene.

5. Use of inhibitors of the mRNA transcripts of the genes listed in Table 1 for the preparation of a therapeutical composition for the treatment of cancer.

6. Use as claimed in claim 5, wherein the inhibitors are antisense molecules, in particular antisense RNA or antisense oligodeoxynucleotides.

7. Use as claimed in claim 5, wherein the inhibitors are double stranded RNA molecules for RNA interference.

8. Use as claimed in claim 1 and claim 5, wherein the treatment comprises gene therapy.

9. Use as claimed in any one of the claims 1-8, wherein the therapeutical composition is for treatment of inflammatory diseases.

10. Use of the murine genes and/or their human homologues listed in Table 1 or their expression products for the diagnosis of cancer.

11. Use as claimed in claim 10, wherein the diagnosis is performed by means of histological analysis of tissue specimens using specific antibodies directed against gene products, and/or in-situ hybridisation analysis of gene expression levels in tissue specimens using specific RNA probes directed against gene sequences.

12. Inhibitor compound directed against the expressed proteins of a murine gene and/or its human homologue listed in Table 1 for use in the treatment of cancer.

13. Inhibitor compound as claimed in claim 12, which is an antibody or derivative thereof directed against the expression products of a gene that is expressed on the cell membrane.

14. Inhibitor compound as claimed in claim 13, wherein the derivative is selected from the group consisting of scFv fragments, Fab fragments, chimeric antibodies, bifunctional antibodies, or other antibody-derived molecules.

15. Inhibitor compound as claimed in claim 12, which is a small molecule interfering with the biological activity of the protein expressed by the gene.

16. Inhibitor compound directed against the transcription product (mRNA) of a murine gene and/or its human homologue listed in Table 1 for use in the treatment of cancer.

17. Inhibitor compound as claimed in claim 16, which is an antisense molecule, in particular an antisense RNA or an antisense oligodeoxynucleotide.

18. Inhibitor compound as claimed in claim 17, which is a double stranded RNA molecule for RNA interference.

19. Diagnostic agent for diagnosing cancers in which one or more of the murine genes and/or their human homologues listed in Table 1 are involved.

20. Diagnostic agent as claimed in claim 19, which is a specific antibody directed against a gene product or a specific RNA probe specific for a gene sequence.

21. Therapeutical composition for the treatment of cancers in which one or more of the murine genes and/or their human homologues listed in Table 1 are involved, comprising a suitable excipient, carrier or diluent and one or more inhibitor compounds as claimed in claims 12-18.

22. Diagnostic composition for the diagnosis of cancers in which one or more of the murine genes and/or their human homologues listed in Table 1 are involved, comprising a suitable excipient, carrier or diluent and one or more diagnostic compounds as claimed in claims 19-20.

23. Compositions as claimed in claim 21 or 22, wherein the cancer is a solid tumor of e.g. lung, colon, breast, prostate, ovarian, pancreas and leukemia.

24. Method for the development of therapeutic inhibitor compounds as claimed in claims 12-18, which method comprises the steps:
a) identification of genes involved in cancer, in particular by using retroviral insertional tagging, optionally in a specific genetic background;
b) validation of one or more of the identified genes as potential target gene(s) for the therapeutic compound by one or more of the following methods:
c) confirmation of the identified gene by Northern Blot analysis in cancer cell-lines;
d) determination of the expression profile of the identified gene in tumors and normal tissue;
e) determination of the functional importance of the identified genes for cancer;
f) production of the expression product of the gene; and
g) use of the expression product of the gene for the production or design of a therapeutic compound.

25. Method as claimed in claim 24, wherein the gene identified in step a) is selected from the murine genes and/or their human homologues listed in Table 1.
